Europäisches Patentamt

European Patent Office

Office européen des brevets

(19)

(11) EP 0 531 647 B1

(12) **EUROPÄISCHE PATENTSCHRIFT**

(45) Veröffentlichungstag und Bekanntmachung des Hinweises auf die Patenterteilung:
**30.10.1996 Patentblatt 1996/44**

(51) Int Cl.⁶: **C07H 15/04**, C07H 1/06 // B01D1/22

(21) Anmeldenummer: **92111468.2**

(22) Anmeldetag: **07.07.1992**

(54) **Verfahren zur destillativen Abtrennung von Fettalkoholen aus fettalkoholischen Alkylpolyglycosid-Lösungen**

Method of distillation separation of fatty alcohols from fatty alcohol alkylpolyglycoside solutions

Méthode de séparation par la distillation d'alcools gras des solutions d'alcool gras alkylpolyglycosidiques

(84) Benannte Vertragsstaaten:
**DE ES FR GB IT**

(30) Priorität: **06.09.1991 DE 4129587**

(43) Veröffentlichungstag der Anmeldung:
**17.03.1993 Patentblatt 1993/11**

(73) Patentinhaber: **HÜLS AKTIENGESELLSCHAFT
D-45764 Marl (DE)**

(72) Erfinder:
• **Müller, Bernd, Dr.
W-4370 Marl (DE)**
• **Ripke, Norbert, Dr.
W-4358 Haltern (DE)**

(56) Entgegenhaltungen:
**EP-A- 0 092 876      EP-A- 0 306 651
EP-A- 0 306 652      EP-A- 0 421 187
WO-A-91/04980**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur destillativen Abtrennung von Fettalkoholen aus fettalkoholischen Alkylpolyglycosid-Lösungen, wobei die Destillation bei 160 bis 200 °C und bei einem Druck von 0,1 bis 20 hPa durchgeführt wird.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylgruppen werden im allgemeinen durch ein- oder mehrstufige Synthesen hergestellt.

Ein einstufiges Herstellverfahren wird u. a. in der deutschen Patentanmeldung mit dem Aktenzeichen P 41 01 252.6 beschrieben.

Ein zweistufiges Verfahren wird beispielsweise in EP-A-0 306 652 angegeben, wonach man zunächst durch Glycosidierung mit n-Butanol ein n-Butylglycosid und daraus durch Umglycosidierung mit einem langkettigen Alkohol das gewünschte langkettige Alkylpolyglycosid herstellt.

Nach beendeter Reaktion liegen die Alkylpolyglycoside in langkettigen Alkoholen gelöst vor. Anschließend müssen diese Alkohole abgetrennt werden, wenn man in Wasser klar lösliche Produkte erhalten will.

Nach EP 0 077 167 soll der Überschuß an Alkohol möglichst schnell entfernt werden. Dabei sollen die Reaktionsprodukte thermisch nicht zu stark belastet werden. In den Beispielen werden die Alkohole im Vakuum destilliert. Die dabei eingesetzten Geräte und die Bedingungen werden aber nicht angegeben.

In EP 0 092 876 werden Fettalkohole aus fettalkoholischen Alkylpolyglycosid-Lösungen mit Hilfe eines Dünnschichtverdampfers destilliert. Dabei soll die Reynolds-Zahl > 20 000 betragen.

Dieses Erfordernis der hohen Reynolds-Zahl führt dazu, daß bei Einsatz hochviskoser Produkte eine hohe Schichtdicke und eine hohe Umdrehungszahl des Rotors im Dünnschichtverdampfer eingestellt werden müssen. Dadurch werden die Verdampfer aber leicht mit Crackprodukten verunreinigt, außerdem werden die beweglichen Teile vorzeitig verschlissen.

Es bestand daher die Aufgabe, auch für hochviskose Alkylpolyglycosid-Lösungen ein Verfahren zur produktschonenden Destillation von Fettalkoholen bereitzustellen.

Die Aufgabe wird erfindungsgemäß dadurch gelöst, daß man die Fettalkohole in einem Dünnschichtverdampfer bei einer Reynolds-Zahl von 30 bis 18 000 abtrennt.

Überraschenderweise ist im laminaren und schwach turbulenten Bereich eine schnelle und produktschonende Destillation möglich.

Die hier eingesetzten Alkylpolyglycoside können nach allen bekannten Methoden, beispielsweise einstufig oder zweistufig durch Glycosidierung und Umglycosidierung, hergestellt werden. Sie enthalten Alkylgruppen mit 10 bis 18 C-Atomen, wobei Alkylgruppen mit 12 bis 16 C-Atomen vorzugsweise vorhanden sind. Der mittlere Polymerisationsgrad liegt bei 1,05 bis 1,4, vorzugsweise bei 1,2 bis 1,4. Die Glycosideinheiten können dabei beispielsweise von Glucose, Mannose, Maltose oder Lactose hergeleitet werden.

Geeignete Fettalkohole weisen 10 bis 18 C-Atome auf, wobei Alkohole mit 12 bis 16 C-Atomen vorzugsweise eingesetzt werden. Geeignet sind beispielsweise Decanol, Dodecanol, Tetradecanol und auch Alkoholgemische. Von diesen Alkoholen leiten sich auch die Alkylgruppen der Alkylpolyglycoside her.

Zum Einsatz kommen 10- bis 70%ige neutral oder schwach alkalisch eingestellte alkoholische Alkylpolyglycosid-Lösungen. Dabei verwendet man vorzugsweise 20- bis 50%ige Lösungen. Geeignete Lösungen erhält man beispielsweise direkt nach der Reaktion mit dem Fettalkohol oder auch nach der Reaktion und nach destillativer Abtrennung von Leichtsiedern.

Geeignete Dünnschichtverdampfer sind beispielsweise Fallfilmverdampfer. Vorzugsweise werden jedoch Dünnschichtverdampfer eingesetzt, mit denen durch rotierende Wischer bestimmte Schichtdicken eingestellt werden können.

Die Reynolds-Zahl kann am Eingang in den Dünnschichtverdampfer an der Alkylpolyglycosid-Lösung oder am Ausgang des Dünnschichtverdampfers an der Alkylpolyglycosid-Schmelze gemessen werden. Die Reynolds-Zahl liegt vorzugsweise im Bereich von 50 bis 12 000.

Die Fettalkohole werden vorzugsweise bei 170 bis 180 °C und bei einem Druck von 0,1 bis 10 hPa abdestilliert.

Nach dem vorliegenden Verfahren werden Fettalkohole bis zu einem Restgehalt von < 2 % bei Verweilzeiten von 15 Sekunden bis 2 Minuten abgetrennt. Das Verfahren ermöglicht die Destillation bei hohem Vakuum und bei relativ niedrigen Temperaturen. Man erhält dabei hellfarbene Alkylpolyglycoside, die klare wäßrige Lösungen ergeben. Wegen der niedrigen Reynolds-Zahl ist die Schaumbildung und dadurch auch die Bildung von festen Ablagerungen im Dünnschichtverdampfer gering.

In den folgenden Beispielen werden neutralisierte alkoholische Alkylpolyglycosid-Lösungen eingesetzt, bei denen die Alkylgruppen der Alkylpolyglycoside den Alkylresten der Alkohole entsprechen.

Die Reynolds-Zahl wird nach der Formel

$$Re = \frac{\delta \cdot u \cdot d}{\eta},$$

wobei $\delta$ = Dichte, $u$ = Umlaufgeschwindigkeit, $d$ = Spaltbreite und $\eta$ = Viskosität sind, bestimmt.

## Beispiel 1

Eine neutralisierte 25%ige Lösung von Alkylpolyglycosiden (mittlerer Polymerisationsgrad: 1,4) in einem Alkoholgemisch aus < 1,5 % Decanol, ca. 68 % Dodecanol, ca. 27 % Tetradecanol, < 6 % Hexadecanol und < 0,5 % Octadecanol (NAFOL[R] 1214 Z, Fa. Condea, D-2212 Brunsbüttel) wird in einen Dünnschichtverdampfer eingebracht. Die Lösung hat bei 170 °C eine Dichte von 777 kg/m³ und eine Viskosität von 3 mPa s.

Das Alkoholgemisch wird in dem Dünnschichtverdampfer bei 170 °C und einem Druck von 1 hPa abdestilliert. Dabei hat der Wischer des Dünnschichtverdampfers eine Umlaufgeschwindigkeit von 8,4 m/s. Die Spaltbreite beträgt 0,0015 m.

Man erhält ein hellfarbenes Produkt, das bei 170 °C eine Dichte von 1 034 kg/m³ und eine Viskosität von 190 mPa s aufweist. Der Restalkoholgehalt liegt bei < 1 %.
Dünnschichtverdampfer-Eingang: Re = 3 263
Dünnschichtverdampfer-Ausgang: Re = 69

## Beispiel 2

Die Alkylpolyglycosid-Lösung von Beispiel 1 wird auf einen Dünnschichtverdampfer gegeben, der bei 180 °C, 1 hPa und mit einer Umlaufgeschwindigkeit von 8,4 m/s und einer Spaltbreite von 0,001 m betrieben wird.

Die eingesetzte Lösung hat bei 180 °C eine Dichte von 769 kg/m³ und eine Viskosität von 2 mPa s.

Man erhält ein hellfarbenes Alkylpolyglycosid mit einem Restalkoholgehalt von < 1 %.
Dünnschichtverdampfer-Eingang: Re = 3 230

## Beispiel 3

Eine neutralisierte 25%ige Lösung von Alkylpolyglycosiden (mittlerer Polymerisationsgrad: 1,4) in einem Alkoholgemisch aus < 2 % Decanol, ca. 70 % Dodecanol, ca. 27 % Tetradecanol und < 1,5 % Hexadecanol (NAFOL[R] 1214 S) wird in einen Dünnschichtverdampfer eingebracht. Die Lösung hat bei 180 °C eine Dichte von 730 kg/m³ und eine Viskosität von 0,9 mPa s.

Das Alkoholgemisch wird im Dünnschichtverdampfer bei 180 °C und 1 hPa sowie bei einer Umlaufgeschwindigkeit des Wischers von 8,4 m/s und einer Spaltbreite von 0,0025 m abdestilliert.

Man erhält ein Alkylpolyglycosid mit einem Restalkoholgehalt von < 1 %.
Dünnschichtverdampfer-Eingang: Re = 17 033

## Beispiel 4

Eine neutralisierte 25%ige Lösung von Alkylpolyglycosiden (mittlerer Polymerisationsgrad: 1,3) in dem Alkoholgemisch von Beispiel 1 wird in einen Dünnschichtverdampfer eingebracht.

Der Dünnschichtverdampfer wird bei 180 °C und 1 hPa betrieben und weist eine Umlaufgeschwindigkeit von 8,4 m/s und eine Spaltbreite von 0,0015 m auf.

Das Produkt am Ausgang des Verdampfers weist bei 180 °C eine Dichte von 1 025 kg/m³ und eine Viskosität von 110 mPa s auf und hat einen Restalkoholgehalt von < 1 %.
Dünnschichtverdampfer-Ausgang: Re = 117

## Patentansprüche

1. Verfahren zur destillativen Abtrennung von Alkoholen aus alkoholischen Alkylpolyglycosid-Lösungen, wobei die Alkohole und die Alkylgruppen der Alkylpolyglycoside 10 bis 18 C-Atome enthalten und der mittlere Polymerisationsgrad 1,05 bis 1,4 beträgt, bei 160 bis 200 °C und 0,1 bis 20 hPa, dadurch gekennzeichnet, daß die Destillation in einem Dünnschichtverdampfer bei einer Reynolds-Zahl von 30 bis 18 000 durchgeführt wird.

2. Verfahren nach Anspruch 1, dadurch gekennzeichnet, daß man 10- bis 70%ige alkoholische Alkylpolyglycosid-Lösungen einsetzt.

3. Verfahren nach Anspruch 1, dadurch gekennzeichnet, daß die Alkohole und die Alkylgruppen der Alkylpolyglycoside 12 bis 16 C-Atome enthalten.

4. Verfahren nach Anspruch 1, dadurch gekennzeichnet, daß die Alkylpolyglycoside mittlere Polymerisationsgrade von 1,2 bis 1,4 aufweisen.

5. Verfahren nach Anspruch 1, dadurch gekennzeichnet, daß die Destillation bei Reynolds-Zahlen von 50 bis 12 000 durchgeführt wird.

## Claims

1. A process for the separation of alcohols by distillation from alcoholic solutions of alkyl polyglycosides at 160 to 200°C and 0.1 to 20 hPa, the alcohols and the alkyl groups of the alkyl polyglycosides having 10 to 18 carbon atoms and the average degree of polymerisation being 1.05 to 1.4, characterised in that the distillation is carried out in a thin-film evaporator at a Reynolds number of 30 to 18,000.

2. A process as claimed in claim 1, characterised in that 10 to 70% alcoholic solutions of alkyl polyglycosides are used.

3. A process as claimed in claim 1, characterised in that the alcohols and the alkyl groups of the alkyl polyglycosides have 12 to 16 carbon atoms.

4. A process as claimed in claim 1, characterised in that the alkyl polyglycosides have an average degree of, polymerisation of 1.2 to 1.4.

5. A process as claimed in claim 1, characterised in that the distillation is carried out at a Reynolds number of 50 to 12,000.

**Revendications**

1. Procédé pour la séparation par distillation à 160-200 °C et 0,1-20 hPa, d'alcools contenus dans des solutions alcooliques de polyglucosides alkylés, les alcools et les groupes alkyle des polyglucosides alkylés contenant 10 à 18 atomes de carbone et le degré moyen de polymérisation étant compris entre 1,05 et 1,4, caractérisé par le fait que l'on réalise la distillation dans un évaporateur à couche mince, avec un nombre de Reynolds compris entre 30 et 18000.

2. Procédé selon la revendication 1, caractérisé par le fait que l'on utilise des solutions alccolisées de polyglucosides alkylés, dosées à 10 - 70 %.

3. Procédé selon la revendication 1, caractérisé par le fait que les alcools et les groupes alkyle des polyglucosides alkylés contiennent 12 à 16 atomes de carbone.

4. Procédé selon la revendication 1, caractérisé par le fait que les polyglucosides alkylés présentent des degrés moyens de polymérisation compris entre 1,2 et 1,4.

5. Procédé selon la revendication 1, caractérisé par le fait que l'on réalise la distillation avec des nombres de Reynolds compris entre 50 et 12000.